# EUROPÄISCHE PATENTANMELDUNG

(11) **EP 0 893 445 A1**
(43) Veröffentlichungstag der Anmeldung: **27.01.1999**
(21) Anmeldenummer: 98112716.0
(22) Anmeldetag: 09.07.1998
(51) Int. Cl.: C07D 491/04

(54) **Verfahren zur Herstellung von 2-Chlorbenzimidazol-Derivaten**

(30) Priorität: 24.07.1997 DE 19731799; 16.10.1997 DE 19745692
(71) Anmelder: BAYER AG, 51368 Leverkusen (DE)
(72) Erfinder: Conrad, Michael, Dr., 42327 Wuppertal (DE); Assmann, Lutz, Dr., 25826 St Peter-Ording (DE); Wroblowsky, Heinz-Jürgen, Dr., 40764 Langenfeld (DE); Casser, Carl, Dr., 51061 Köln (DE); Bielefeldt, Dietmar, Dr., 40883 Ratingen (DE)

(57) **Zusammenfassung**

Nach einem neuen Verfahren lassen sich 2-Chlor-benzimidazol-Derivate der Formel 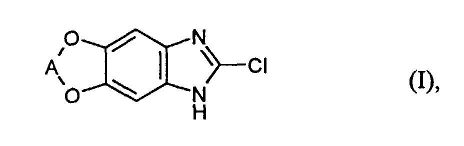 in welcher
- A: für gegebenenfalls halogeniertes Alkylen mit 1 oder 2 Kohlenstoffatomen steht,
herstellen, indem man 1,3-Dihydro-benzimidazol(thi)one der Formel 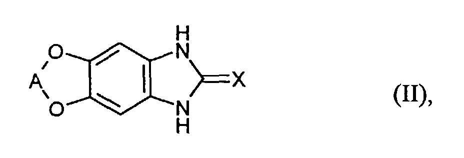 in welcher
- X: für Sauerstoff oder Schwefel steht,
mit Phosphoroxychlorid umsetzt.

Neue 1,3-Dihydro-benzimidazol(thi)one der Formel (II) und Verfahren zu deren Herstellung.

## Beschreibung

Die vorliegende Erfindung betrifft ein neues Verfahren zur Herstellung von bekannten 2-Chlor-benzimidazol-Derivaten, die als Zwischenprodukte zur Synthese von Wirkstoffen mit mikrobiziden Eigenschaften verwendbar sind. Außerdem betrifft die Erfindung neue 1,3-Dihydro-benzimidazol(thi)one und ein Verfahren zu deren Herstellung.

Es ist bereits bekannt geworden, daß sich in 2-Position chlorierte Benzimidazol-Derivate herstellen lassen, indem man die entsprechenden 2-Brom-imidazole mit Chlorwasserstoff umsetzt (vgl. WO-A 97-06171). So erhält man zum Beispiel durch Behandlung von 2-Brom-6,6-difluor-[1.3]dioxolo-[4,5-f]benzimidazol mit Chlorwasserstoffgas in Gegenwart von Dimethylformamid das 2-Chlor-6,6-difluor-[1,3]dioxolo-[4,5-f]-benzimidazol. Nachteilig an diesem Verfahren ist aber, daß die gewünschten Produkte in relativ niedrigen Ausbeuten anfallen.

Außerdem ist schon bekannt, daß sich 2-Chlor-benzimidazol herstellen läßt, indem man 1,3-Dihydro-benzimidazol-2-on mit Phosphoroxychlorid in Gegenwart von Chlorwasserstoff umsetzt (vgl. J. Chem. Soc. 1963, 2930). Entsprechende Chlorierungen von 1,3-Dihydro-benzimidazol-2-onen, die am Benzolring noch einen weiteren annellierten, heterocyclischen Ring enthalten, wurden aber bisher noch nicht beschrieben.

Es wurde nun gefunden, daß man 2-Chlor-benzimidazol-Derivate der Formel 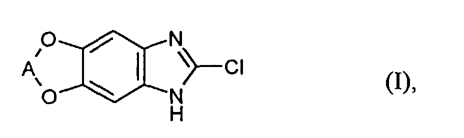 in welcher
- A: für gegebenenfalls halogeniertes Alkylen mit 1 oder 2 Kohlenstoffatomen steht,
erhält, indem man 1,3-Dihydro-benzimidazol(thi)one der Formel 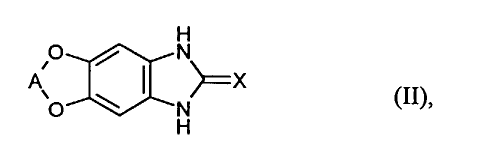 in welcher
- A: die oben angegebene Bedeutung hat und
- X: für Sauerstoff oder Schwefel steht,
mit Phosphoroxychlorid, gegebenenfalls in Gegenwart von Chlorwasserstoff oder von Phosphorpentachlorid, sowie gegebenenfalls in Gegenwart eines Verdünnungsmittels bei Temperaturen zwischen 50°C und 150°C umsetzt.

Es ist als ausgesprochen überraschend zu bezeichnen, daß sich 2-Chlor-benzimidazol-Derivate der Formel (I) nach dem erfindungsgemäßen Verfahren in wesentlich höherer Ausbeute herstellen lassen als nach der bisher bekannten Methode. Unerwartet ist auch, daß speziell Phosphoroxychlorid besonders gut zur Chlorierung von 1,3-Dihydro-benzimidazol(thi)onen der Formel (II) geeignet ist.

Das erfindungsgemäße Verfahren zeichnet sich durch eine Reihe von Vorteilen aus. So ermöglicht es, wie schon erwähnt, die Synthese von 2-Chlor-benzimidazol-Derivaten der Formel (I) in sehr hoher Ausbeute. Günstig ist auch, daß die benötigten Reaktionskomponenten einfach herstellbar und auch in größeren Mengen verfügbar sind. Ein weiterer Vorteil besteht schließlich darin, daß die Durchführung der Umsetzung und die Isolierung der Reaktionsprodukte keinerlei Schwierigkeiten bereitet.

Verwendet man 6,6-Difluor-[1,3]dioxolo-[4,5-f]-1,3-dihydro-benzimidazol-2-on als Ausgangssubstanz und Phosphoroxychlorid in Gegenwart von gasförmigem Chlorwasserstoff als Chlorierungsmittel, so kann der Verlauf des erfindungsgemäßen Verfahrens durch das folgende Formelschema veranschaulicht werden.

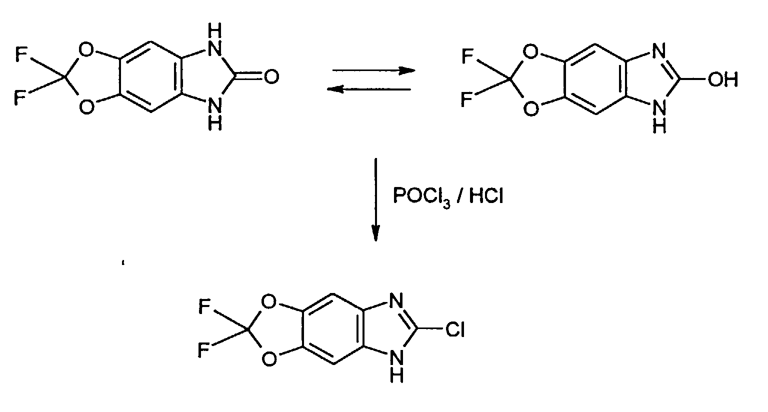

Die bei der Durchführung des erfindungsgemäßen Verfahrens als Ausgangsstoffe benötigten 1,3-Dihydro-benzimidazol(thi)one sind durch die Formel (II) allgemein definiert. Bevorzugt sind Verbindungen der Formel (II), in welcher
- A: für gegebenenfalls einfach oder zweifach durch Fluor und/oder Chlor substituiertes Methylen steht oder für gegebenenfalls einfach bis vierfach durch Fluor und/oder Chlor substituiertes Ethylen steht und
- X: für Sauerstoff oder Schwefel steht.

Besonders bevorzugt sind 1,3-Dihydro-benzimidazol(thi)one der Formel (II), in welcher
- A: für die Gruppen -CH₂-, -CF₂-, -CCl₂-, -CF₂-CF₂-, -CHF-CF₂, -CHF-CHF-, -CF₂-CFCl- oder -CFCl-CFCl- steht und
- X: für Sauerstoff oder Schwefel steht.

Die 1,3-Dihydro-benzimidazol(thi)one der Formel (II) sind bisher noch nicht bekannt. Sie lassen sich herstellen, indem man Phenylendiamine der Formel 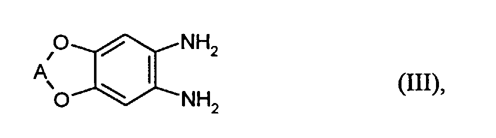 in welcher
- A: die oben angegebene Bedeutung hat, entweder

a) mit Phosgen der Formel 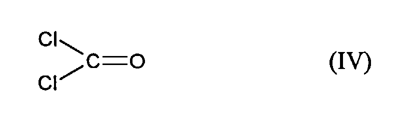 in Gegenwart eines Verdünnungsmittels bei Temperaturen zwischen 10°C und 120°C umsetzt, oder
b) mit 1,1-Carbonyl-diimidazol der Formel 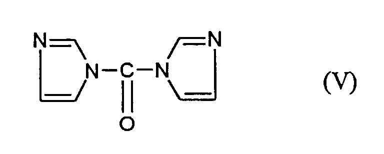 in Gegenwart eines Verdünnungsmittels bei Temperaturen zwischen 10°C und 80°C umsetzt,
   oder
c) mit Harnstoff der Formel 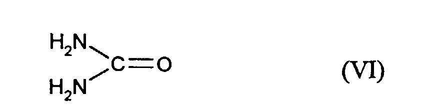 gegebenenfalls in Gegenwart eines Verdünnungsmittels bei Temperaturen zwischen 130°C und 200°C umsetzt, oder
d) mit Kalium-ethyl-xanthogenat der Formel 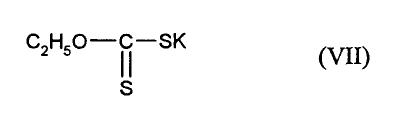 in Gegenwart eines Verdünnungsmittels bei Temperaturen zwischen 20°C und 100°C umsetzt.

Verwendet man 5,6-Diamino-2,2-difluor-benzodioxol und Phosgen als Ausgangsstoffe, so kann der Verlauf des obigen Verfahrens durch das folgende Formelschema veranschaulicht werden.

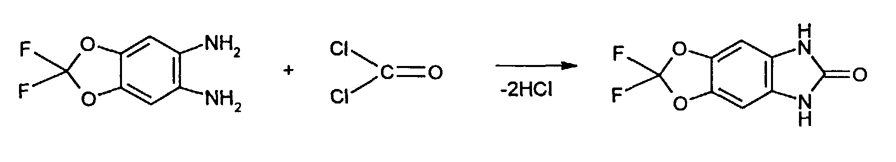

Die bei der Durchführung des obigen Verfahrens zur Herstellung von 1,3-Dihydro-benzimidazol(thi)onen als Ausgangsstoffe benötigten Phenylendiamine sind durch die Formel (III) allgemein definiert. Bevorzugt sind Verbindungen der Formel (III), in denen A diejenigen Bedeutungen hat, die bereits im Zusammenhang mit der Beschreibung der 1,3-Dihydro-benzimidazol(thi)one der Formel (II) für A als bevorzugt genannt wurden.

Die Phenylendiamine der Formel (III) sind bekannt oder lassen sich nach bekannten Verfahren herstellen (vgl. DE-A 36 05 977 und WO 97-06171).

Die bei der Durchführung des obigen Verfahrens zur Herstellung von 1,3-Dihydro-benzimidazol(thi)onen der Formel (II) nach den Varianten (a) bis (d) als Reaktionskomponenten benötigten Verbindungen der Formeln (IV) bis (VII) sind bekannt.

Als Verdünnungsmittel kommen bei der Durchführung der Variante (a) des obigen Verfahrens inerte anorganische und organische Solventien in Betracht. Vorzugsweise verwendbar sind Wasser, wäßrige Salzsäure und halogenierte aliphatische oder aromatische Kohlenwasserstoffe, wie Methylenchlorid, Chloroform, Tetrachlorkohlenstoff oder Chlorbenzol.

Die Reaktionstemperaturen können bei der Durchführung der Variante (a) des obigen Verfahrens innerhalb eines größeren Bereiches variiert werden. Im allgemeinen arbeitet man bei Temperaturen zwischen 10°C und 120°C, vorzugsweise zwischen 20°C und 110°C.

Bei der Durchführung der Variante (a) des obigen Verfahrens arbeitet man im allgemeinen unter Atmosphärendruck. Es ist jedoch auch möglich, unter erhöhtem Druck zu arbeiten.

Bei der Durchführung der Variante (a) des obigen Verfahrens setzt man auf 1 mol an Phenylendiamin der Formel (III), vorzugsweise 1 bis 5 mol an Phosgen der Formel (IV) ein. Die Aufarbeitung erfolgt nach üblichen Methoden. Im allgemeinen geht man so vor, daß man nach beendeter Phosgenierung den anfallenden Feststoff abfiltriert, wäscht und trocknet.

Als Verdünnungsmittel kommen bei der Durchführung der Variante (b) des obigen Verfahrens übliche inerte, organische Solventien in Betracht. Vorzugsweise verwendbar sind Ether, wie Diethylether, Diisopropylether, Methyl-tert.-butylether, Methyltert.-amylether, Dioxan, Tetrahydrofuran, 1,2-Dimethoxyethan, 1,2-Diethoxyethan oder Anisol.

Die Reaktionstemperaturen können bei der Durchführung der Variante (b) des obigen Verfahrens in einem bestimmten Bereich variiert werden. Im allgemeinen arbeitet man bei Temperaturen zwischen 10°C und 80°C, vorzugsweise zwischen 20°C und 70°C.

Bei der Durchführung der Variante (b) des obigen Verfahrens arbeitet man im allgemeinen unter Atmosphärendruck. Es ist aber auch möglich, unter erhöhtem Druck zu arbeiten.

Bei der Durchführung der Variante (b) des obigen Verfahrens setzt man auf 1 mol an Phenylendiamin der Formel (III) im allgemeinen eine äquivalente Menge oder auch einen Überschuß, vorzugsweise 1,1 bis 1,5 mol an 1,1-Carbonyl-diimidazol der Formel (V) ein. Die Aufarbeitung erfolgt nach üblichen Methoden. Im allgemeinen verfährt man in der Weise, daß man das Reaktionsgemisch auf verdünnte, wäßrige Mineralsäure gibt, mehrfach mit einem mit Wasser wenig mischbaren organischen Solvens extrahiert, die vereinigten organischen Phasen wäscht, trocknet und unter vermindertem Druck einengt. Das verbleibende Produkt kann nach üblichen Methoden, wie Umkristallisation oder Chromatographie, von eventuell noch vorhandenen Verunreinigungen befreit werden.

Bei der Durchführung der Variante (c) des obigen Verfahrens arbeitet man vorzugsweise in der Schmelze in Abwesenheit von zusätzlichen Verdünnungsmitteln. Die Reaktionstemperaturen können dabei innerhalb eines bestimmten Bereiches variiert werden. Im allgemeinen arbeitet man bei Temperaturen zwischen 130°C und 200°C, vorzugsweise zwischen 140°C und 190°C.

Es ist aber auch möglich, bei der Durchführung der Variante (c) des obigen Verfahrens in Gegenwart von Verdünnungsmitteln, wie zum Beispiel Dimethylformamid oder Diethylenglykol, zu arbeiten.

Bei der Durchführung der Variante (c) des obigen Verfahrens arbeitet man im allgemeinen unter Atmosphärendruck. Es ist aber auch möglich, unter erhöhtem oder vermindertem Druck zu arbeiten.

Bei der Durchführung der Variante (c) des obigen Verfahrens setzt man auf 1 mol an Phenylendiamin der Formel (III) im allgemeinen eine äquivalente Menge oder auch einen Überschuß, vorzugsweise 2 bis 4 mol an Harnstoff der Formel (VI) ein. Die Aufarbeitung erfolgt nach üblichen Methoden. Im allgemeinen geht man so vor, daß man das Reaktionsgemisch mit verdünnter, wäßriger Alkalimetallhydroxid-Lösung versetzt, das entstehende Gemisch filtriert, das Filtrat ansäuert und den dabei anfallenden Niederschlag abfiltriert, wäscht und trocknet.

Als Verdünnungsmittel kommen bei der Durchführung der Variante (d) des obigen Verfahrens übliche inerte, organische Solventien in Frage. Vorzugsweise verwendbar sind Alkohole, wie Methanol, Ethanol, n-Propanol oder Isopropanol.

Die Reaktionstemperaturen können auch bei der Durchführung der Variante (d) des obigen Verfahrens im einem größeren Bereich variiert werden. Im allgemeinen arbeitet man bei Temperaturen zwischen 20°C und 100°C, vorzugsweise zwischen 30°C und 90°C.

Auch bei der Durchführung der Variante (d) des obigen Verfahrens arbeitet man im allgemeinen unter Atmosphärendruck. Es ist aber auch möglich, unter erhöhtem oder vermindertem Druck zu arbeiten.

Bei der Durchführung der Variante (d) des obigen Verfahrens setzt man auf 1 mol an Phenylendiamin der Formel (III) im allgemeinen eine äquivalente Menge oder auch einen Überschuß, vorzugsweise 1,1 bis 1,5 mol an Kalium-ethyl-xanthogenat der Formel (VII) ein. Die Aufarbeitung erfolgt wiederum nach üblichen Methoden. Dabei geht man im allgemeinen so vor, daß man das Reaktionsgemisch, gegebenenfalls nach vorherigem Versetzen mit Aktivkohle, filtriert, das Filtrat mit Wasser versetzt und ansäuert, und den anfallenden Niederschlag absaugt, wäscht und trocknet.

Als Reaktionskomponente bei der Durchführung des erfindungsgemäßen Verfahrens dient Phosphoroxychlorid, gegebenenfalls in Gegenwart von Chlorwasserstoff oder von Phosphorpentachlorid.

Als Verdünnungsmittel kommen bei der Durchführung des erfindungsgemäßen Verfahrens Phosphoroxychlorid und halogenierte aliphatische oder aromatische Kohlenwasserstoffe, wie Dichlormethan, Chloroform, Tetrachlormethan, Dichlorethan, Trichlorethan, Chlorbenzol oder Dichlorbenzol, in Frage. Vorzugsweise verwendet man Phosphoroxychlorid sowohl als Reaktionskomponente als auch als Verdünnungsmittel.

Die Reaktionstemperaturen können bei der Durchführung des erfindungsgemäßen Verfahrens innerhalb eines größeren Bereiches variiert werden. Im allgemeinen arbeitet man bei Temperaturen zwischen 50°C und 150°C, vorzugsweise zwischen 70°C und 130°C.

Bei der Durchführung des erfindungsgemäßen Verfahrens arbeitet man im allgemeinen unter Atmosphärendruck. Es ist aber auch möglich, unter erhöhtem Druck zu arbeiten. In einer bevorzugten Variante des erfindungsgemäßen Verfahrens arbeitet man in einem verschlossenen Druckgefäß unter dem sich einstellenden Eigendruck.

Bei der Durchführung des erfindungsgemäßen Verfahrens setzt man auf 1 Mol an 1,3-Dihydro-benzimidazol(thi)on der Formel (II) im allgemeinen einen so hohen Überschuß an Phosphoroxychlorid ein, daß es sowohl als Reaktionskomponente als auch als Verdünnungsmittel fungiert. Außerdem leitet man gegebenenfalls gasförmigen Chlorwasserstoff in das Reaktionsgemisch oder man fügt Phosphorpentoxid hinzu. Die Aufarbeitung erfolgt nach üblichen Methoden. Im allgemeinen geht man so vor, daß man das Reaktionsgemisch unter vermindertem Druck einengt, den Rückstand mit Eis oder Wasser versetzt und den anfallenden Niederschlag absaugt, wäscht und trocknet. Das saure Filtrat wird durch Zugabe von wäßriger Base neutralisiert, und der dabei anfallende Niederschlag wird ebenfalls abgesaugt, gewaschen und getrocknet.

Die nach dem erfindungsgemäßen Verfahren herstellbaren 2-Chlor-benzimidazol-Derivate der Formel (I) sind als wertvolle Zwischenprodukte zur Synthese von Wirkstoffen mit mikrobiziden, vorzugsweise fungiziden Eigenschaften bekannt (vgl. WO-A 97-06 171). So lassen sich fungizid wirksame Chlorbenzimidazole der Formel 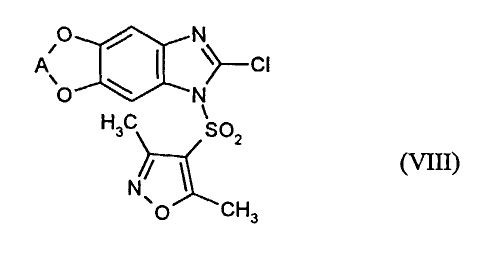 in welcher
- A: die obengenannte Bedeutung hat,
herstellen, indem man 2-Chlorbenzimidazol-Derivate der Formel 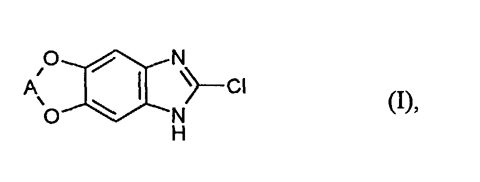 in welcher
- A: die oben angegebene Bedeutung hat,
mit 3,5-Dimethyl-isoxazol-4-sulfonylchlorid der Formel 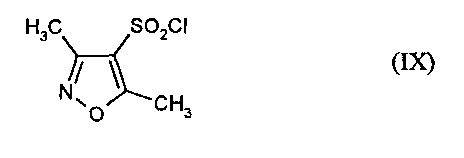 in Gegenwart eines Säurebindemittels, wie Natriumhydrid oder Kaliumcarbonat, und in Gegenwart eines Verdünnungsmittels, wie Tetrahydrofuran oder Acetonitril umsetzt.

Die Durchführung des erfindungsgemäßen Verfahrens wird durch die folgenden Beispiele veranschaulicht.

### Herstellungsbeispiele

### Beispiel 1

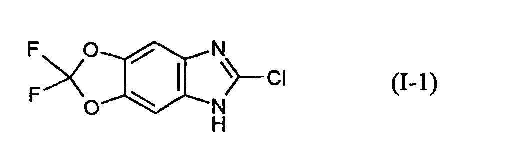

Ein Gemisch aus 10 g (44 mmol) 6,6-Difluor-[1,3]dioxolo-[4,5-f]-1,3-dihydro-benzimidazol-2-on und 100 ml frisch destilliertem Phosphoroxychlorid wird 15 Minuten unter Rückfluß erhitzt. Danach erhitzt man weitere 6 Stunden unter Rückfluß und leitet dabei Chlorwasserstoffgas mit einer Geschwindigkeit von etwa 8 Blasen pro Sekunde ein. Anschließend wird das Reaktionsgemisch unter vermindertem Druck soweit eingeengt, daß es noch gut rührbar ist. Man versetzt den Rückstand mit 100 g Eis und rührt 16 Stunden bei 5°C. Der angefallene Rückstand wird abgesaugt, mit Wasser gewaschen und über Phosphorpentoxid getrocknet. Man erhält 3,5 g eines Produktes, das im wesentlichen aus 2-Chlor-6,6-difluor-[1,3]dioxolo[4,5-f]benzimidazol besteht.

Das saure Filtrat wird unter Kühlung mit konzentrierter Ammoniak-Lösung neutralisiert. Das entstehende Gemisch wird 40 Minuten bei 5°C gerührt. Der dabei anfallende Feststoff wird abgesaugt, mit Wasser gewaschen und über Phosphorpentoxid getrocknet. Man erhält 9 g eines Produktes, das zu 80 % aus 2-Chlor-6,6-difluor[1,3]dioxolo[4,5-f]-benzimidazol besteht.

Danach errechnet sich eine Gesamtausbeute von 85 % der Theorie an 2-Chlor-6,6-difluor[1,3]dioxolo[4,5-f]-benzimidazol.

### Beispiel 2

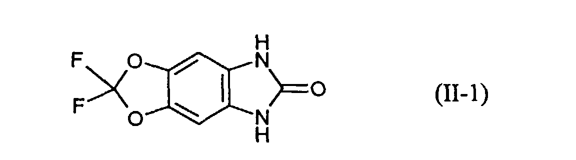

In ein Gemisch aus 9,4 kg (46,2 Mol) 5,6-Diamino-2,2-difluor-benzodioxol und 47 kg Wasser werden unter Rühren bei Raumtemperatur zunächst 5,0 kg Phosgen in einer Dosierrate von 1,7 kg/h und dann weitere 3,5 kg Phosgen in einer Dosierrate von 0,6 kg/h eingeleitet, wobei die Temperatur des Reaktionsgemisches auf 45°C ansteigt. Man rührt das Reaktionsgemisch 13 Stunden bei dieser Temperatur und fügt dann noch 0,34 kg Phosgen hinzu. Nach dem Abkühlen des Reaktionsgemisches auf Raumtemperatur und nach Spülen des Reaktionsgefäßes mit Stickstoff werden zur Neutralisation 15,8 kg an 45 gew.-%iger wäßriger Natronlauge hinzugegeben. Der anfallende Niederschlag wird abgesaugt, portionsweise mit insgesamt 50 kg Wasser gewaschen und unter vermindertem Druck bei einer Temperatur von 60°C getrocknet. Man erhält auf diese Weise 9,85 kg (91,8 % der Theorie) an 6,6-Difluor-[1,3]dioxolo-[4,5-f]-1,3-dihydro-benzimidazol-2-on in Form einer Festsubstanz mit einem Schmelzpunkt von über 220°C.
¹H-NMR-Spektrum (DMSO, TMS):
   δ = 7,05 ppm (s, 2H, CH)
   δ = 10,8 ppm (s, 2H, NH)

### Beispiel 3

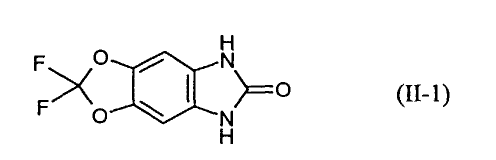

In eine Lösung aus 0,8 Mol 5,6-Diamino-2,2-difluor-benzodioxol, 600 ml Wasser und 250 ml 37 %iger, wäßriger Salzsäure werden unter Rühren bei 30 bis 40°C innerhalb von einer Stunde 150 g Phosgen eingeleitet. Man läßt eine Stunde bei 30 bis 40°C nachrühren, spült dann das Reaktionsgefäß mit Stickstoff und filtriert den angefallenen Feststoff ab. Das Produkt wird mit Wasser gewaschen und danach getrocknet. Man erhält auf diese Weise 160 g (92 % der Theorie) an 6,6-Difluor-[1,3]-dioxolo-[4,5-f]-1,3-dihydro-benzimidazol-2-on in Form einer Festsubstanz mit einem Schmelzpunkt von über 220°C.

### Beispiel 4

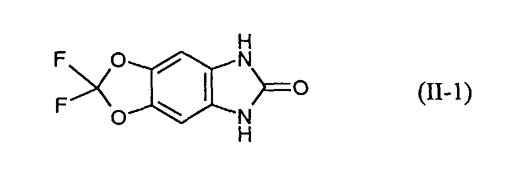

In ein Gemisch von 20,8 g (0,13 Mol) 1,1-Carbonyl-diimidazol und 150 ml Tetrahydrofuran werden bei Raumtemperatur unter Rühren 18,8 g (0,1 Mol) 5,6-Diamino-2,2-difluor-benzodioxol gegeben, wobei die Reaktionstemperatur leicht ansteigt. Das Reaktionsgemisch wird 18 Stunden bei Raumtemperatur gerührt und dann auf 600 ml 1N Schwefelsäure gegeben. Das entstehende Gemisch wird mehrfach mit insgesamt 300 ml Diethylether extrahiert. Die vereinigten organischen Phasen werden mit 200 ml Wasser gewaschen, über Natriumsulfat getrocknet und unter vermindertem Druck eingeengt. Der verbleibende Rückstand wird mit 50 ml Petrolether verrührt. Der anfallende Niederschlag wird abgesaugt und getrocknet. Man erhält auf diese Weise 19,3 g eines Produktes, das gemäß HPLC-Analyse zu 84 % aus 6,6-Difluor-[1,3]dioxolo-[4,5-f]-1,3-dihydro-benzimidazol-2-on besteht. Danach errechnet sich eine Ausbeute von 76 % der Theorie.
Schmelzpunkt >220°C.

### Beispiel 5

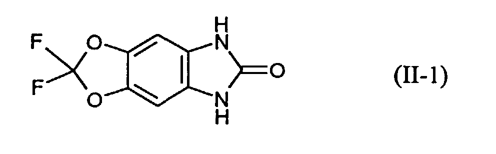

In eine Schmelze von 30 g (0,5 Mol) Harnstoff wird unter Rühren bei 140°C ein Gemisch aus 94 g (0,5 Mol) 5,6-Diamino-2,2-difluor-benzodioxol und 60 g (1 Mol) Harnstoff innerhalb von 30 Minuten portionsweise gegeben. Die Schmelze wird weitere 3 Stunden bei 170°C gerührt, dann abgekühlt auf 70°C und mit 600 ml 2N wäßriger Natronlauge versetzt. Man rührt 30 Minuten und saugt dann den angefallenen Niederschlag ab. Das Filtrat wird mit Essigsäure angesäuert. Der dabei anfallende Niederschlag wird abgesaugt, mit Wasser gewaschen und getrocknet. Man erhält auf diese Weise 89,5 g an 6,6-Difluor-[1,3]dioxolo-[4,5-f]-1,3-dihydro-benzimidazol-2-on. Die Ausbeute errechnet sich danach zu 83 % der Theorie.

### Beispiel 6

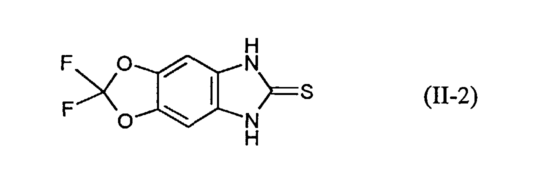

Ein Gemisch aus 18,8 g (0,1 Mol) 5,6-Diamino-2,2-difluor-benzodioxol, 18,5 g (0,11 Mol) Kalium-ethyl-xanthogenat, 100 ml Ethanol und 15 ml Wasser wird 3 Stunden unter Rückfluß erhitzt. Nach dem Abkühlen auf Raumtemperatur bläst man Stickstoff durch das Reaktionsgefäß und versetzt dann mit 4 g Aktivkohle. Man erhitzt das Gemisch 15 Minuten unter Rückfluß, saugt heiß ab und wäscht mit heißem Ethanol nach. Das Filtrat wird auf 60 bis 70°C erwärmt und mit 100 ml Wasser von ebenfalls 60 bis 70°C versetzt. Anschließend wird eine Lösung von 8,3 ml (0,13 Mol) Eisessig in 16,7 ml Wasser hinzugegeben. Das Gemisch wird 16 Stunden bei 4°C stehengelassen. Der entstandene Niederschlag wird abgesaugt, mit kaltem Wasser gewaschen und über Phosphorpentoxid getrocknet. Man erhält auf diese Weise 18,1 g eines Produktes, das gemäß HPLC-Analyse zu 78,34 % aus 6,6-Difluor-[1,3]dioxolo-[4,5-f]-1,3-dihydrobenzimidazol-2-thion besteht. Die Ausbeute errechnet sich danach zu 62 % der Theorie.

### Beispiel 7

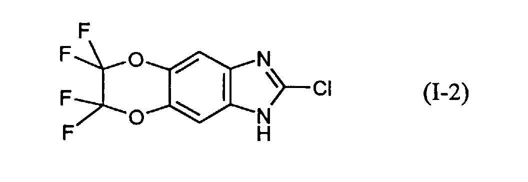

Ein Gemisch aus 10,6 g (40 mmol) 6,6,7,7-Tetrafluor-[1,4]dioxino-[2,3-f]-1,3-dihydro-benzimidazol-2-on und 60 ml Phosphoroxychlorid wird 20 Stunden unter Rückfluß erhitzt. Danach wird das Reaktionsgemisch auf 600 ml Eis gegeben. Das entstehende Gemisch wird zweimal mit je 150 ml Diethylether extrahiert. Die vereinigten organischen Phasen werden mit Wasser gewaschen, über Natriumsulfat getrocknet und unter vermindertem Druck eingeengt. Das erhaltene Produkt wird mit Diethylether an Kieselgel chromatographiert. Man erhält auf diese Weise 4,1 g einer Festsubstanz, die gemäß HPLC-Analyse zu 82 % aus 2-Chlor-6,6,7,7-tetrafluor-[1,4]dioxino[2,3-f]-benzimidazol besteht. Die Ausbeute errechnet sich danach zu 30 % der Theorie.

### Beispiel 8

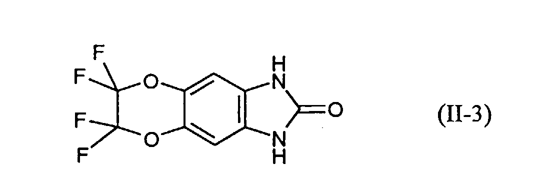

In ein Gemisch aus 16,0 g (0,1 Mol) 1,1-Carbonyl-diimidazol und 150 ml Tetrahydrofuran werden bei Raumtemperatur unter Rühren 23,8 g (0,1 Mol) 6,7-Diamino-2,2,3,3-tetrafluor-benzodioxin gegeben, wobei die Temperatur des Reaktionsgemisches leicht ansteigt. Das Reaktionsgemisch wird 18 Stunden bei Raumtemperatur gerührt und dann auf 600 ml 1N Schwefelsäure gegeben. Das entstehende Gemisch wird mehrfach mit insgesamt 300 ml Diethylether extrahiert. Die vereinigten organischen Phasen werden mit 100 ml Wasser gewaschen, über Natriumsulfat getrocknet und unter vermindertem Druck eingeengt. Der verbleibende Rückstand wird mit 30 ml Petrolether und 10 ml Diethylether verrührt. Der anfallende Niederschlag wird abgesaugt und getrocknet. Man erhält auf diese Weise 20,7 g eines Produktes, das gemäß HPLC-Analyse zu 75 % aus 6,6,7,7-Tetrafluor-[1,4]dioxino-[2,3-f]-1,3-dihydro-benzimidazol-2-on besteht. Die Ausbeute errechnet sich danach zu 59 % der Theorie.

### Beispiel 9

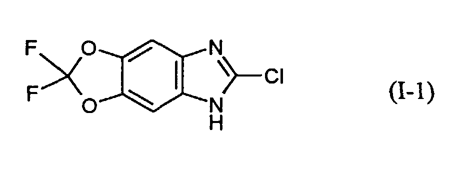

Ein Gemisch aus 10 g (44 mmol) 6,6-Difluor-[1,3]-dioxolo-[4,5-f]-1,3-dihydrobenzimidazol-2-on und 100 ml frisch destilliertem Phosphoroxychlorid werden in einem verschlossenen Druckgefäß 6 Stunden auf 120°C erhitzt, wobei sich ein Eigendruck von etwa 2 bar einstellt. Anschließend wird das Reaktionsgemisch unter vermindertem Druck eingeengt, mit Eis versetzt und durch Zugabe von wäßrigem Ammoniak neutralisiert. Der angefallene Rückstand wird abgesaugt, mit Wasser gewaschen und über Phosphorpentoxid getrocknet. Man erhält 10,35 g eines Produktes, das gemäß HPLC-Analyse zu 95,6 % aus 2-Chlor-6,6-difluor-[1,3]-dioxolo-[4,5-f]-benzimidazol besteht. Die Ausbeute errechnet sich danach zu 90 % der Theorie.

### Vergleichsbeispiel A

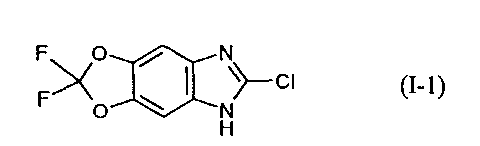

In eine Lösung von 2,8 g (10 mmol) 2-Brom-6,6-difluor-[1,3]dioxolo[4,5-f]benz-imidazol in 30 ml Dimethylformamid wird 2 Stunden lang bei 120°C Chlorwasserstoffgas eingeleitet. Die Mischung wird auf 200 g Eis/Wasser gegeben und dreimal mit je 80 ml Essigsäureethylester extrahiert. Die vereinigten organischen Extrakte werden über Natriumsulfat getrocknet und unter vermindertem Druck eingeengt. Der Rückstand wird mit Diethylether an Kieselgel chromatographiert. Man erhält 0,5 g (21,5 % der Theorie) 2-Chlor-6,6-difluor-[1,3]dioxolo[4,5-f]benzimidazol als weißen Feststoff vom Schmelzpunkt >220°C.

### Verwendungsbeispiel I

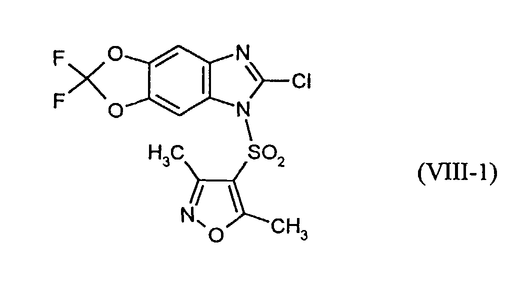

Ein Gemisch aus 96,6 g (0,4 Mol) 2-Chlor-6,6-difluor-[1,3]dioxolo[4,5-f]benz-imidazol und 600 ml Acetonitril wird bei Raumtemperatur unter Rühren mit 81,6 g (0,6 Mol) pulverisiertem Kaliumcarbonat versetzt und 10 Minuten bei Raumtemperatur gerührt. Anschließend fügt man 79,2 g (0,4 Mol) 3,5-Dimethyl-isoxazol-4-sulfonylchlorid hinzu und rührt weitere 20 Stunden bei Raumtemperatur. Das Reaktionsgemisch wird auf 2 Liter Wasser gegossen. Das entstehende Gemisch wird dreimal mit je 500 ml Methylenchlorid extrahiert. Die vereinigten organischen Phasen werden über Natriumsulfat getrocknet und unter vermindertem Druck eingeengt. Der verbleibende Rückstand wird mit Methylenchlorid als Laufmittel an Kieselgel chromatographiert. Man erhält auf diese Weise 117 g (75 % der Theorie) an 1-(3,5-Dimethyl-isoxazol-4-sulfonyl)-2-chlor-6,6-difluor-[1,3]dioxolo-[4,5-f]-benzimidazol in Form einer farblosen Festsubstanz vom Schmelzpunkt 128 bis 131°C.

## Patentansprüche

1. Verfahren zur Herstellung von 2-Chlor-benzimidazol-Derivaten der Formel 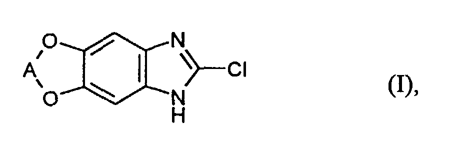 in welcher
A für gegebenenfalls halogeniertes Alkylen mit 1 oder 2 Kohlenstoffatomen steht,
dadurch gekennzeichnet, daß man 1,3-Dihydro-benzimidazol(thi)one der Formel 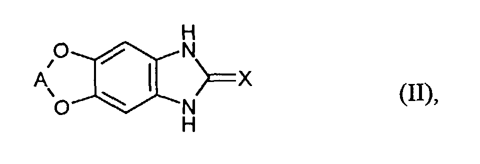 in welcher
A die oben angegebene Bedeutung hat und
X für Sauerstoff oder Schwefel steht,
mit Phosphoroxychlorid, gegebenenfalls in Gegenwart von Chlorwasserstoff oder von Phosphorpentachlorid sowie gegebenenfalls in Gegenwart eines Verdünnungsmittels bei Temperaturen zwischen 50°C und 150°C umsetzt.

2. Verfahren gemäß Anspruch 1, dadurch gekennzeichnet, daß man als Ausgangsstoffe 1,3-Dihydro-benzimidazol(thi)one der Formel (II) einsetzt, in welcher
A für gegebenenfalls einfach oder zweifach durch Fluor und/oder Chlor substituiertes Methylen steht, oder für gegebenenfalls einfach bis vierfach durch Fluor und/oder Chlor substituiertes Ethylen steht und
X für Sauerstoff oder Schwefel steht.

3. Verfahren gemäß Anspruch 1, dadurch gekennzeichnet, daß man als Ausgangsstoffe 1,3-Dihydro-benzimidazol(thi)one der Formel (II) einsetzt, in welcher
A für die Gruppen -CH₂-, -CF₂-, -CCl₂-, -CF₂-CF₂-, -CHF-CF₂, -CHF-CHF-, -CF₂-CFCl- oder -CFCl-CFCl- steht und
X für Sauerstoff oder Schwefel steht.

4. Verfahren gemäß Anspruch 1, dadurch gekennzeichnet, daß man als Ausgangsstoff 6,6-Difluor-[1,3]-dioxolo-[4,5-f]-dihydro-benzimidazol-2-on der Formel 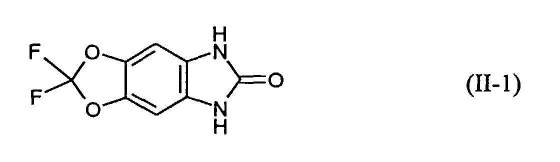 einsetzt.

5. Verfahren gemäß Anspruch 1, dadurch gekennzeichnet, daß man die Umsetzung in einem verschlossenen Druckgefäß unter dem sich einstellenden Eigendruck durchführt.

6. 1,3-Dihydro-benzimidazol(thi)one der Formel 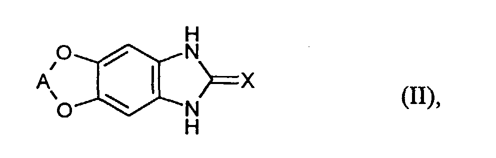 in welcher
A für gegebenenfalls halogeniertes Alkylen mit 1 oder 2 Kohlenstoffatomen steht und
X für Sauerstoff oder Schwefel steht.

7. 1,3-Dihydro-benzimidazol(thi)one der Formel (II) gemäß Anspruch 6, in denen
A für gegebenenfalls einfach oder zweifach durch Fluor und/oder Chlor substituiertes Methylen steht, oder für gegebenenfalls einfach bis vierfach durch Fluor und/oder Chlor substituiertes Ethylen steht und
X für Sauerstoff oder Schwefel steht.

8. 1,3-Dihydro-benzimidazol(thi)one der Formel (II) gemäß Anspruch 6, in denen
A für die Gruppen -CH₂-, -CF₂-, -CCl₂-, -CF₂-CF₂-, -CHF-CF₂, -CHF-CHF-, -CF₂-CFCl- oder -CFCl-CFCl- steht und
X für Sauerstoff oder Schwefel steht.

9. 1,3-Dihydro-benzimidazolon gemäß Anspruch 6, gekennzeichnet durch die Formel

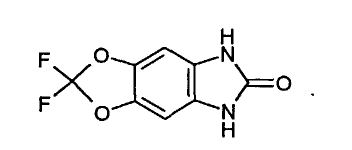

10. Verfahren zur Herstellung von 1,3-Dihydro-benzimidazol(thi)onen der Formel (II) gemäß Anspruch 6, dadurch gekennzeichnet, daß man Phenylendiamine der Formel 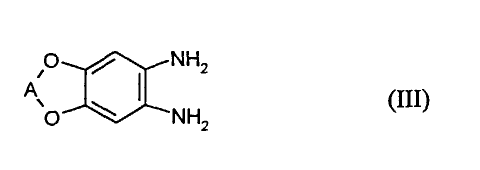 in welcher
A die oben angegebene Bedeutung hat, entweder
a) mit Phosgen der Formel 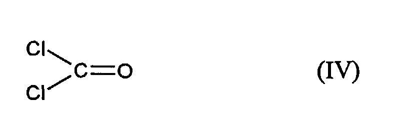 in Gegenwart eines Verdünnungsmittels bei Temperaturen zwischen 10°C und 120°C umsetzt, oder
b) mit 1,1-Carbonyl-diimidazol der Formel 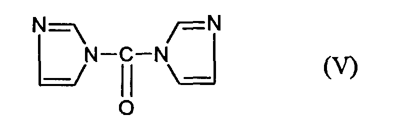 in Gegenwart eines Verdünnungsmittels bei Temperaturen zwischen 10°C und 80°C umsetzt,
oder
c) mit Harnstoff der Formel 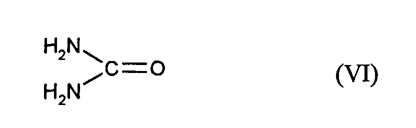 gegebenenfalls in Gegenwart eines Verdünnungsmittels bei Temperaturen zwischen 130°C und 200°C umsetzt, oder
d) mit Kalium-ethyl-xanthogenat der Formel 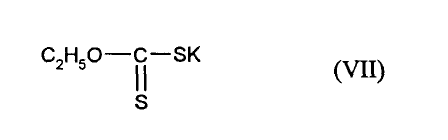 in Gegenwart eines Verdünnungsmittels bei Temperaturen zwischen 20°C und 100°C umsetzt.
